# EUROPEAN PATENT SPECIFICATION

(11) **EP 2 668 918 B1**
(45) Date of publication and mention of the grant of the patent: **05.11.2014**
(21) Application number: 12169894.8
(22) Date of filing: 29.05.2012
(51) Int. Cl.: A61B 17/70

(54) **Receiving part for receiving a rod for coupling the rod to a bone anchoring element and a bone anchoring device with such a receiving part**
Aufnahmeteil für die Aufnahme einer Stange zur Kopplung der Stange in einem Knochenverankerungselement und eine Knochenverankerungsvorrichtung mit einem derartigen Aufnahmeteil
Pièce de réception pour recevoir une tige pour coupler la tige sur un élément d'ancrage d'os et dispositif d'ancrage d'os avec une telle pièce de réception

(43) Date of publication of application: 04.12.2013
(73) Proprietor: Biedermann Technologies GmbH & Co. KG, 78166 Donaueschingen (DE)
(72) Inventor: Biedermann, Lutz, 78048 VS-Villingen (DE); Biedermann, Timo, 78647 Trossingen (DE); Matthis, Wilfried, 79367 Weisweil (DE); Dannecker, Berthold, 78112 St. Georgen (DE)
(74) Representative: Prüfer & Partner GbR European Patent Attorneys

(56) References cited:
- US-A1- 2008 015 580
- US-A1- 2010 152 949
- US-A1- 2010 234 902

## Description

The invention relates to a receiving part for receiving a rod for coupling the rod to a bone anchoring element, wherein the receiving part includes a receiving part body with a channel for receiving the rod and an accommodation space for accommodating a head of the bone anchoring element and a pressure element with a flexible portion to clamp the head. The flexible portion comprises a first slit provided in a circumferential direction with respect to a head of the bone anchoring element and a second slit extending from an open end of the pressure element into the first slit so that a slit ring is integrally formed in the pressure element. Such a receiving part together with a multitude of different bone anchoring elements may constitue a modular polyaxial bone anchoring system.

Various designs of polyaxial bone screws, wherein the head is clamped from the side to lock the angular position of the bone screw, are known.

US 5,672,176 describes a bone screw with a receiving part with a conically shaped seat and a conically shaped pressure element which exerts a pressure onto the head from above and from the side. If the cone angle has a value laying within a specific range self-locking of the pressure element within the receiving part takes place which allows to preliminary lock the head within a receiving part while the rod is still movable in order to allow the adjustment of its position.

US 6,063,090 relates to a device used to connect a longitudinal support to a pedicle screw by an accommodating head having a channel to accommodate the longitudinal support. The pedicle screw and the accommodating head are connected via a conical collet chuck in the accommodating head and by a spherical head on the pedicle screw. The device allows engagement of the pedicle screw in the accommodating head after the pedicle screw has been inserted into the bone.

US 2010/0234902 A1 describes a receiving part for receiving a rod for coupling the rod to a bone anchoring element wherein the receiving part comprises a receiving part body and a pressure element arranged therein. The pressure element is movable along a longitudinal axis of the receiving part body from an inserting position insert the head, to a pre-locking position to clamp the head in the receiving part by a pre-stress exerted by the pressure element, to a locking position to lock the head in the receiving part.

US 2011/0152949 A1 describes a receiving part having an axis of symmetry passing through a top end and a bottom end thereof. The receiving part has a U-shaped recess for receiving a rod and an inner bore including a section with an enlarged diameter and a narrowing section narrowing towards the bottom end and accommodating a head of a bone anchoring element. A pressure element may be provided in the bore, the pressure element having a section at its bottom end which is flexible enough to snap onto the head, when the head is inserted, by virtue a plurality of slits extending from an opening at the bottom end.

It is the object of the invention to provide an improved receiving part for receiving a rod for coupling the rod to a bone anchoring element and a bone anchoring device with such a receiving part, which consists of few parts and has an improved handling during surgery.

The object is solved by a receiving part according to claim 1 and a bone anchoring device according to claim 17. Further developments are given in the dependent claims.

The receiving part comprises a pressure element that has a slit ring at its bottom end. The slit ring is integrally connected to the pressure element, which means the slit ring and the remainder of the pressure element are a monolithic piece. The slit ring can expand in radial direction to allow the insertion of the head of the bone anchor. The force necessary for introducing the head into the flexible portion of the pressure element is reduced compared to known pressure elements that have only longitudinal, i.e. coaxial slits.

Furthermore, the distance that is necessary for moving the pressure element in an axial direction until the expansion of the slit ring allows the insertion of the head of the bone anchor is reduced. This allows and facilitates an in situ mounting procedure of the receiving part to a bone anchor that has already been inserted into the bone.

By means of the elasticity of the slit ring, the head of the bone anchoring element can be clamped by friction between the head and the slit ring. The friction fit between the head of the bone anchor and the pressure element allows a preliminary holding of the bone anchor relative to the receiving part in a desired angular position of the bone anchor. This can be changed by overcoming the friction force as long as the bone anchor is not finally locked with respect to the receiving part.

The receiving part and the bone anchor can be easily disassembled using a simple tool.

Furthermore, the polyaxial bone anchoring device has only few parts that reduces the costs for manufacturing and facilitates handling. Because the slit ring is integrally formed in the pressure element it can not get lost. The pressure element may be secured in the receiving part body against falling out.

The polyaxial bone anchoring device that includes the receiving part allows to provide a modular system wherein various bone anchors and/or different receiving parts are available and can be assembled on demand prior to use.

Further features and advantages of the invention will become apparent from the description of embodiments using the accompanying drawings. In the drawings:
- Fig. 1:: shows a perspective exploded view of a first embodiment of the bone anchoring device.
- Fig. 2:: shows a perspective view of the bone anchoring device of Fig. 1 in an assembled state.
- Fig. 3:: shows a cross-sectional view of the bone anchoring device according to the first embodiment of Fig. 2, the section taken perpendicular to the rod axis.
- Fig. 4:: shows a perspective view of the pressure element.
- Fig. 5:: is a side view of the pressure element.
- Fig. 6:: shows a top view of the pressure element.
- Fig. 7:: shows a cross-sectional view of the pressure element, the section taken along line A-A of Fig. 6.
- Fig. 8:: shows a bottom view of the pressure element.
- Figs. 9 to 14:: show steps of assembling the bone anchoring element and the receiving part of the bone anchoring device according to the first embodiment.
- Figs. 15 and 16:: show two positions of the pressure element in the receiving part with inserted bone anchoring element.
- Fig. 17:: shows a step of disassembling the receiving part and the bone anchor using a tool.
- Fig. 18:: shows a perspective exploded view of a bone anchoring device according to a second embodiment.
- Fig. 19:: shows a perspective view of the pressure element of the bone anchoring device according to the second embodiment.
- Fig. 20:: shows a side view of the pressure element according to the second embodiment.
- Fig. 21:: shows a cross-sectional view of the pressure element according to Fig. 19.
- Figs. 22 and 23:: show steps of assembling and locking with respect to the bone anchoring device according to the second embodiment.
- Fig. 24:: shows a perspective view of a further embodiment of the pressure element of the bone anchoring device.
- Fig. 25:: shows the pressure element shown in Fig. 24 from a bottom perspective view.
- Fig. 26:: shows a side view of the pressure element of Fig. 24.
- Fig. 27:: shows a top view of the pressure element of Fig. 24.
- Fig. 28:: shows a bottom view of the pressure element of Fig. 24.

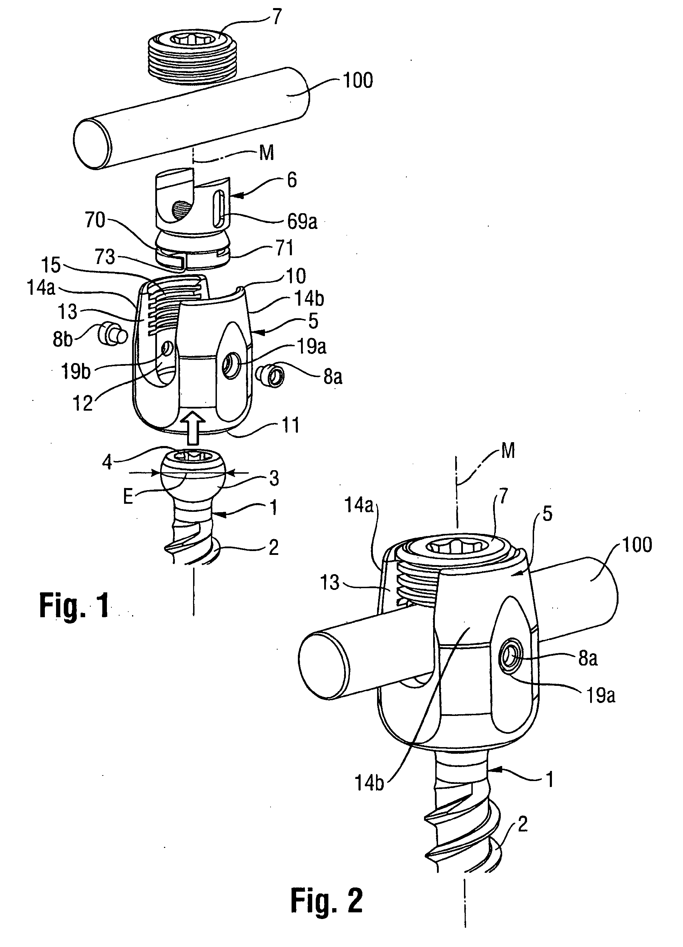

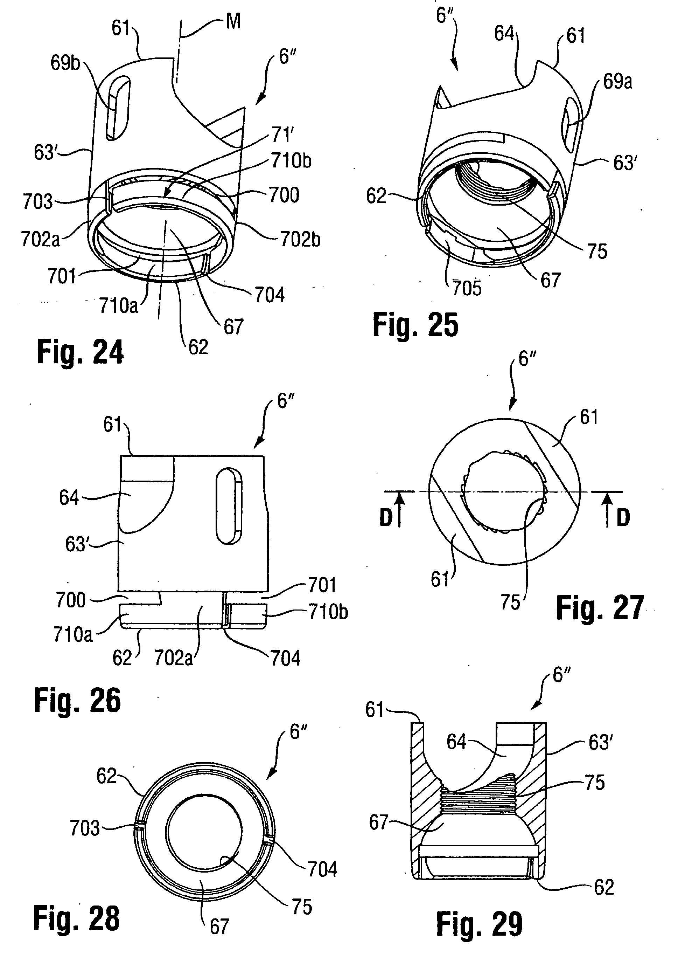

- Fig. 29:: shows a cross-sectional view of the pressure element of Fig. 24 along line D-D shown in Fig. 27. As shown in Figs. 1 and 2, a bone anchoring device according to a first embodiment comprises a bone anchoring element 1 in the form of a bone screw having a threaded shaft 2 and a head 3. The head 3 has a spherical outer surface portion including the greatest outer diameter E of the sphere and a flat free end with a recess 4 for engagement with a screwing-in tool. The bone anchoring device further comprises a receiving part for receiving a stabilization rod 100 and for coupling the stabilization rod 100 to the bone anchoring element 1. The receiving part consists of a receiving part body 5 and a pressure element 6 arranged in the receiving part body 5. The pressure element 6 serves for locking the head 3 in the receiving part body 5. Pins 8 may be provided for holding the pressure element 6 in the receiving part body 5.

Further, a closure element 7 in the form of an inner screw is provided for securing the rod 100 in the receiving part body 5.

The receiving part body 5 will now be explained with reference to Figs. 1 to 3. It comprises a top end 10 and an opposite bottom end 11 and an axis of symmetry M passing through the top end and the bottom end. A bore 12 is provided that is coaxial to the axis of symmetry M. In a first region adjacent the top end 10, the receiving part body 5 has a U-shaped recess 13 with a bottom directed towards the bottom end 11 and two free lateral legs 14a, 14b extending towards the top end 10. In the region of legs 14a, 14b an internal thread 15 is provided that cooperates with the inner screw 7. The channel formed by the U-shaped recess 13 is sized so as to receive the rod 100 therein that connects a plurality of bone anchoring devices. In the region of the legs 14a, 14b, a first portion of the bore 12 comprises a first inner diameter. In a region below the legs 14a, 14b the bore 12 has a widened portion 12a with a diameter greater than the diameter of the first portion. Between the bottom end 11 and the widened portion 12a, the bore 12 comprises a narrowing portion 12b that tapers towards the bottom end 11 with a cone angle. An opening 16 is provided at the bottom end 11, the diameter of the opening 16 being larger than the diameter of the head 3 to allow the introduction of the head 3 from the bottom end 11 of the receiving part. The widened portion 12a and the narrowing portion 12b provide an accommodation space 17 for the head 3 of the bone anchor.

On each leg 14a, 14b, bores 19a, 19b extending through the legs in a direction substantially perpendicular to the central axis M are provided for receiving the pins 8a, 8b. The bores 19a, 19b are located approximately at a center of each leg. The pins 8a, 8b have preferably such a length that once inserted into the bores 19a, 19b, they extend a short distance into the bore 12 to provide a stop for the pressure element 6 described below. The pins 8a, 8b may be flush with the outer surface of the receiving part body 5.
As can be seen in particular in Figs. 1 and 4 to 8, the pressure element 6 has a top end 61 and an opposite bottom end 62. Adjacent the top end 61 there is a substantially cylindrical portion 63 which has an outer diameter that is slightly smaller than the inner diameter of the bore 12, so that the pressure element 6 is moveable in the bore 12. There is a U-shaped recess 64 that is open to the top end 61 for receiving the rod 100 therein when the pressure element 6 is arranged in the receiving part body 5 such that the U-shaped recess 64 is aligned with the U-shaped recess 13 of the receiving part body 5. Adjacent the cylindrical portion 63, a recessed portion 65 is provided that continues into a head receiving portion 66.

The head receiving portion 66 comprises a hollow interior chamber 67 that is substantially spherically-shaped and configured to accommodate the spherical head 3 therein. At the bottom end 62, the head receiving portion 66 has an opening to allow the introduction of the head 3. In a region adjacent the recessed portion 65, the head receiving portion 66 has a spherical outer surface portion that continues into a tapered outer surface portion in the direction towards the second end 62. The taper corresponds substantially to the paper of the narrowing portion 12b of the receiving part.

At a distance from the bottom end 62 a circumferentially extending first slit 70 is provided. The first slit 70 is substantially perpendicular to the central axis M of the receiving part body when the pressure element is arranged in the receiving part body 5. The first slit 70 extends to around more than 180 °, preferably more than 270° and further preferably more than 340° around the central axis M. Hence, by means of the first slit 70, a ring-shaped portion 71 at the bottom end 62 is provided that is integrally connected to the pressure element as a whole by a wall portion forming a connecting strip 72.

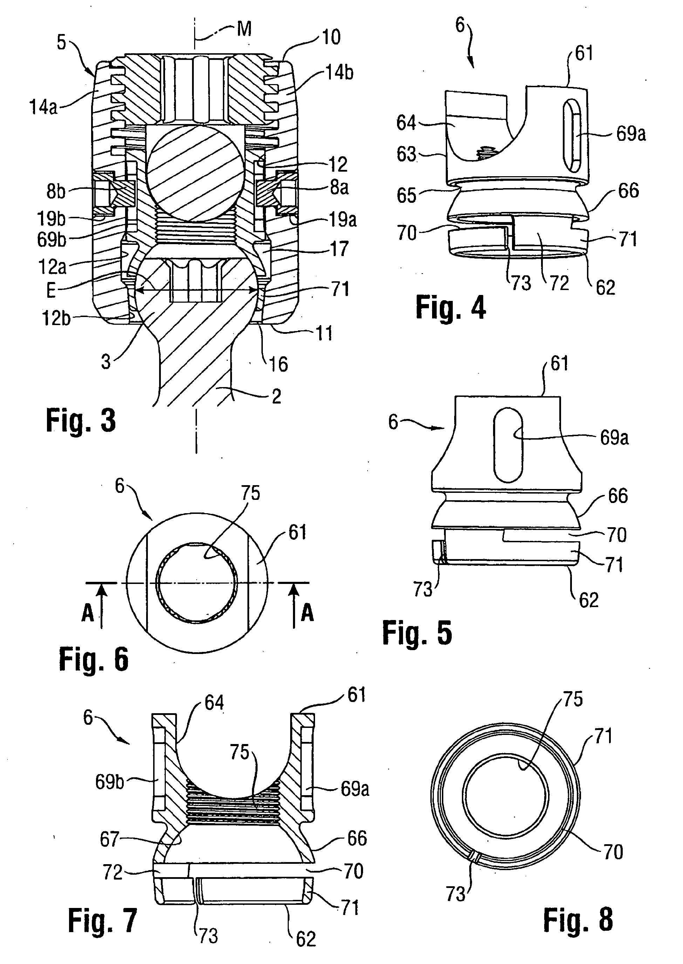

The connecting strip 72 has such a length in a circumferential direction that it provides a stable connection of the ring-shaped portion 71 to the pressure element 6. At one end of the circumferentially extending first slit 70, there is a coaxially extending second slit 73 that extends from the second end 62 fully through the ring-shaped portion 71 into the first slit 70. By means of this, the ring-shaped portion is cut through in circumferential direction and forms a slit ring 71 that can be expanded and compressed in the radial direction. The width of the vertical second slit 73 is preferably smaller than the width of the circumferential first slit 70. This can be seen in particular in Figs. 3 and 7. The outer surface of the slit ring 71 is tapered towards the bottom end 62 of the pressure element 6. The connecting strip 72 and the vertical second slit 73 are arranged at an angle relative to the U-shaped recess 64. The position and size of the slit ring 71 is such that when the head 3 of the bone anchoring element is inserted from the open bottom end 62 of the pressure element, the slit ring 71 expands in that the width of the vertical slit becomes larger, and when the head 3 has been fully inserted into the hollow interior chamber 67, the slit ring encompasses the head 3 at or below the greatest diameter E of the head 3 in the direction to the shaft 2.

The maximum outer diameter of the head receiving portion 66 is slightly smaller than the inner diameter of the bore 12 in the first portion and is smaller than the diameter of the bore in the widened portion 12a. Hence, the slit ring 71 is configured to expand in the widened portion 12a of the receiving part body 5.

At the lateral sides of the U-shaped recess, the pressure element comprises two elongate recesses 69a, 69b that are elongate in the direction from the top end to the bottom end. The recesses 69a, 69b are provided in the outer wall. These elongate recesses 69a, 69b serve for engagement by the pins 8a, 8b, respectively, when the pressure element is inserted and provide for a stop when the pins abuts against one of the ends of the recesses in axial direction.

Finally, the pressure element 6 comprises a coaxial through hole 75 allowing access to the recess 4 of the head 3 of the bone anchor with a tool (not shown). The through hole 75 is preferably provided in at least a portion thereof with an internal thread as shown in Figs. 3 and 7. The internal thread serves for engagement with a tool to fix the pressure element when head 3 of the bone anchor shall be removed from the head receiving portion 66 to be described below.

The pressure element 6 may be arranged in the receiving part by introduction from the top end 10 of the receiving part and by aligning the U-shaped recesses of the pressure element and the receiving part body. The pins 8a, 8b may be inserted thereafter to extend into the elongate recesses 69a, 69b of the pressure element to hold the pressure element within the receiving part thereby preventing escaping of the pressure element through the first end 10. By means of this the pressure element and the receiving part body are pre-assembled and can be connected to a bone anchoring element.

All parts described before may be made of a bio-compatible material, such as a bio-compatible metal like stainless steel or titanium, or a bio-compatible metal alloy, e.g., Nitinol, or are made from a bio-compatible plastic material, e.g. Polyetheretherketon (PEEK). The parts may be all of the same material or of different materials.

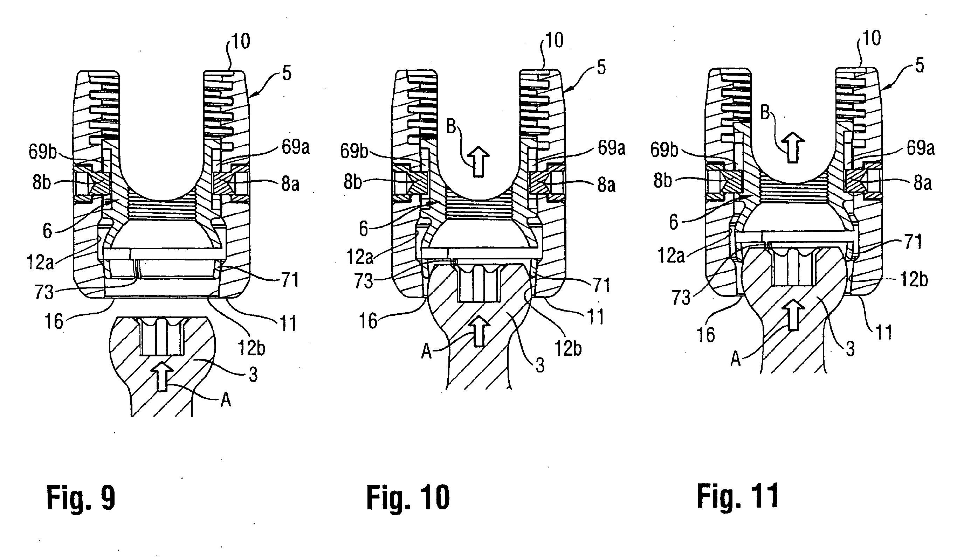

Steps of assembling the bone anchoring device will now be explained with reference to Figs. 9 to 14. As shown in Fig. 9, the pressure element and the receiving part body 5 are pre-assembled as described above. The slit ring 71 abuts against the upper part of the narrowing portion 12b. The bone anchoring element 1 is introduced from the bottom end 11 of the receiving part body (arrow A). Next, as shown in Fig. 10, the head 3 of the bone anchoring element is introduced from the bottom end 11 and touches the slit ring 71 of the pressure element 6, thereby shifting the pressure element in the direction of the arrow B until the lower end of the elongate recesses 69a, 69b abut against the pins 8a, 8b, respectively, as shown in Fig. 11. In this portion, the slit ring 71 is situated in the accommodation space provided by the widened portion 12a of the receiving part body 5.

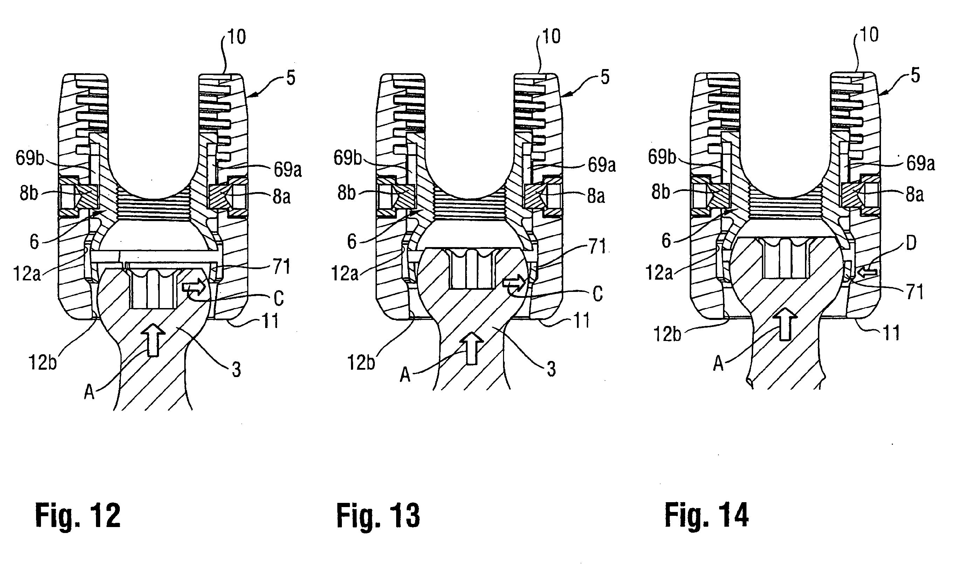

Further introduction of the head 3 into the head receiving portion 66 expands the slit ring 71 within the widened portion 12a of the receiving part body 5. The head 3 can be inserted. Because the slit ring 71 does not expand at the connecting strip 72, the introduction of the head 3 is not precisely coaxially to the axis of symmetry M but is slightly out of the axis of symmetry M. By the further introduction of the head 3 the slit ring 71 is expanded to the maximum extent as shown in Fig. 13. Still further introduction of the head 3 allows the head 3 to enter the upper space of the hollow interior chamber 67 until it abuts against the inner wall of the chamber 67. Thereby the slit ring 71 elastically contracts around the head 3as shown in Fig. 14.

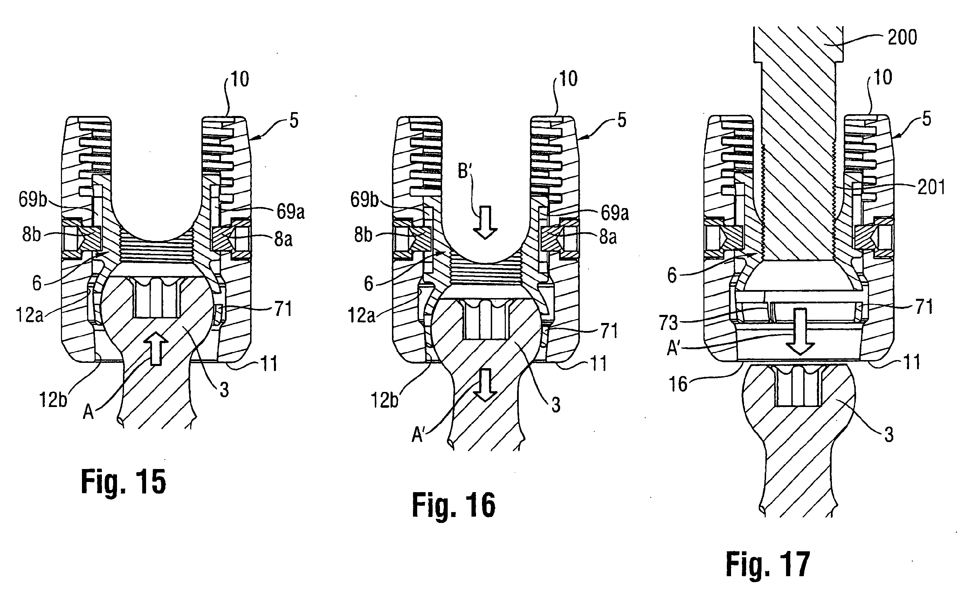

When the head 3 abuts against the inner wall of the head receiving portion 66 in the upper region, the slit ring 71 encompasses the portion of the head below the equator E as can be seen in Fig. 15. In this condition, the slit ring 71 frictionally clamps the head 3 so that the bone anchoring element can be pivoted to a desired angular position and held there by the friction fit between the pressure element 6 and the head 3.

Finally, moving down the pressure element 6 presses the slit ring 71 into the narrowing portion 12b so that the slit ring 71 locks the head as shown in Fig. 16.

In clinical use, first the receiving part may be assembled with the bone anchoring element as described above, then the bone anchoring element may be inserted into the bone. Usually at least two bone anchoring devices are inserted into the bone and the receiving parts are aligned. Then, the rod is inserted into the receiving part 9. Finally, the inner screw (closure element) 7 is screwed between the legs 14a, 14b of the device and the rod with the pressure element is pressed down so that the head 3 is locked.

Alternatively, the bone anchoring element can be inserted first into the bone and the receiving part body 5 with the pressure element 6 can be mounted thereafter onto the head 3 of the bone anchoring element. Because of the slit ring 71 the force necessary to insert the head into the head receiving portion 66 of the pressure element is reduced and the distance needed to move the pressure element within the bore 12 of the receiving part body is reduced.

In the case that the receiving part body 5 and the head 3 shall be disassembled, i.e. the head 3 shall be removed from the receiving part body 5, a tool 200 may be used as shown in Fig. 17. The tool 200 may have a first portion to be gripped by a person (not shown) and a threaded end portion 201 that engages the threaded through hole 75 of the pressure element 6. By means of this, the pressure element 6 can be pulled into the direction of the top end 10 of the receiving part body until the pins 8a, 8b abut against the lower end of the elongate recesses 69a, 69b. In this position, the slit ring 71 is located within the accommodation space in the widened portion 12a of the receiving part body. Then, the head 3 can be removed through the bottom end 11 of the receiving part body 5.

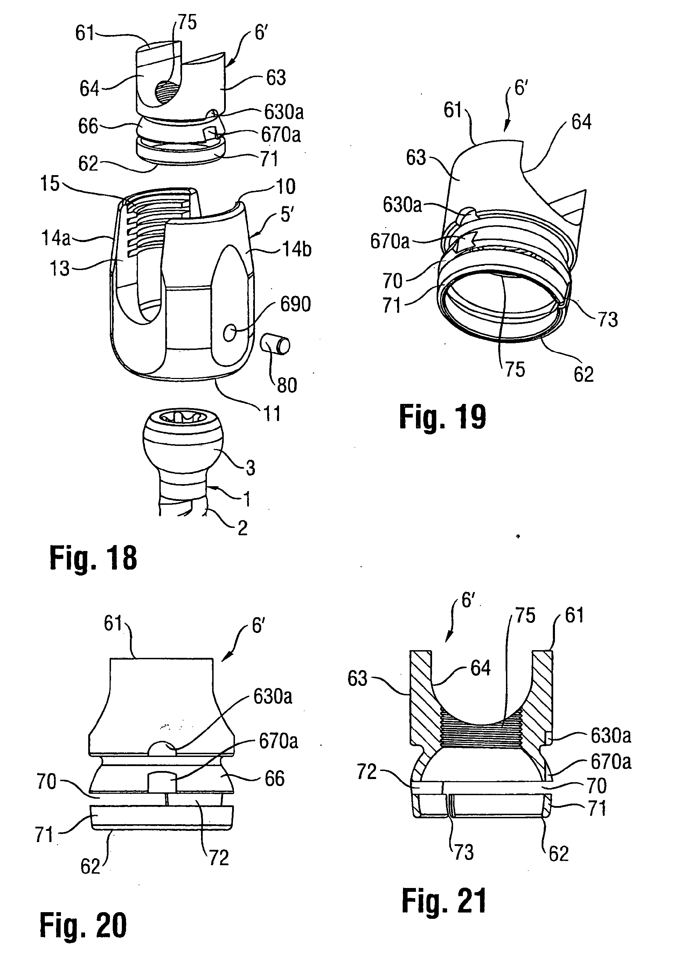

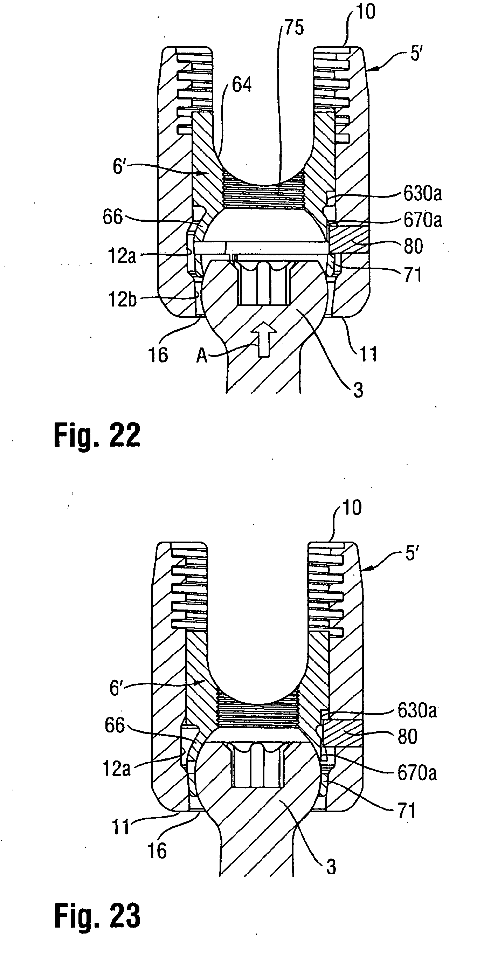

A second embodiment of the bone anchoring device is shown in Figs. 18 to 23. With reference to Fig. 18, the bone anchoring device according to the second embodiment differs from the bone anchoring device according to the first embodiment in that the receiving part body 5' has only one lateral bore 690 provided in the leg 14a at a distance below the bottom of the U-shaped recess 13. Hence, as shown in Figs. 22 and 23, a single pin 80 extends through the bore 690 into the widened portion 12a of the bore 12. All other parts of the receiving part body are identical to the first embodiment and the description thereof will not be repeated.

The pressure element 6' comprises at the end of the cylindrical portion 63 a first substantially semi-circular notch 630a that is open towards the bottom end 62 and configured to engage with the pin 80 as shown in Fig. 23. Hence, the notch 630a is provided at 90° to the U-shaped recess 64.

The pressure element 6' further comprises a second notch 670a that is provided in an axial direction below the first notch 630a in the upper part of the head receiving portion 66. The width of the second notch 670a is slightly larger than the diameter of the pin 80 so that the pin 80 can engage the second notch 670a. As can be seen in particular in Fig. 21, the second notch 670a has such a depth that it is recessed with respect to the slit ring 71. The notches 630a, 670a are arranged on the opposite side of the connection strip 72, as best seen in Figs. 20 and 21.

In use, the pressure element 6' and the receiving part body 5' are pre-assembled. The head 3 is introduced like in the first embodiment from the bottom end 11 . When the head 3 is introduced and abuts against the bottom end of the receiving part, the slit ring 71 abuts against the pin 80 as shown in Fig. 22. Therefore, the pin 80 provides a stop for the slit ring 71 and prevents bending of the slit ring in a direction towards the top end 10 of the receiving part body 5. Simultaneously, the pin 80 acts as a securing device against escaping of the pressure element 6' and as positioning device for a correct alignment of the pressure element 6' with respect to the receiving part body 5'.

A further embodiment of the pressure element 6" will be described with reference to Figs. 24 to 29. The pressure element 6" has as in the previous embodiments, a top end 61 and a bottom end 62. Adjacent the top end 61, a substantially cylindrical portion 63' is provided that extends to a distance from the bottom end 62 and comprises adjacent the top end 61 the U-shaped recess 64. A central bore 75 with a threaded portion is present as in the previous embodiments.

At a distance from the bottom end 62 two slits 700, 701 are provided that extend in a transverse direction relative to the central axis M. At the end of each of the horizontal slits 700, 70 1 a vertical slit 703, 704 is provided, wherein the vertical slits are located at the circumference of the pressure element on opposite places. The vertical slits 703, 704 extend from the bottom end 62 fully into the horizontal slits 700, 703, respectively. Hence, two ring portions 710a, 710b that are opposite to each form the flexible section of the pressure element. The ring portions 710a, 710b are connected with the cylindrical portion 63' via connecting strips 702a, 702b located opposite to each other. By means of the two ring portions 710a, 710b a slit ring 71' is formed. The slit ring 71' can expand, wherein the vertical slits 703, 704 are enlarged and can elastically return to its non-expanded condition.

Because the pressure element is almost not flexible in the region of the connecting strips 702a, 702 b a recess 705 may be provided on one or both of the inner wall portions of the connecting strips 702a, 702b such that the inner diameter of the pressure element in the region of the connecting strips 702a, 702b is the same or slightly larger than the largest outer diameter E of the head 3.

Further, the pressure element comprises the interior chamber 67 with a preferably spherically-shaped section to accommodate the head 3.

The pressure element 63' can be combined with a receiving part body according to the first and second embodiment. The features of the embodiments can be combined among each other. The pressure element can be further modified, for example, more than two horizontal and two vertical slits may be provided.

Further modifications of the embodiment shown are possible. For example, the circumferential slit 70, 700, 701 needs not to be perpendicular to the central axis, it can also be inclined. The vertical slit 73, 703, 704 can also be inclined or can have a particular contour, as long as it extends fully through the ring. The head of the bone anchoring element can have any other shape, e.g., a cylindrical shape wherein a monoaxial bone screw is provided allowing rotation of the screw element with respect to the receiving part around a single axis. The head 3 can also be, e.g., conically shaped. The hollow interior chamber is then adapted to the shape of the head. In a further modification, the receiving part body can have an inclined bottom end to allow a greater angulation of the head in one direction.

In another modification, the narrowing portion 12d of the receiving part body 5 in the corresponding outer surface portion of the slit ring 71,71' are not tapered but spherically curved.

For the bone anchoring element, all kinds of bone anchoring elements can be used, such as screws of different length, of different diameter, of different threadforms, nails with barbs or without barbs, cannulated screws or nails, or hooks.

For the closure element all known kinds of closure elements can be used, single part closure elements, double part closure elements etc.

## Claims

1. A receiving part for receiving a rod (100) for coupling the rod to a bone anchoring element (1), the receiving part including
a receiving part body (5, 5') with a top end (10), a bottom end (11) and a central axis (M) extending through the top end and the bottom end;
a channel (13) for receiving the rod, and
an accommodation space (12a, 12b) for accommodating a head (3) of the bone anchoring element, the accommodation space having an opening (16) at the bottom end for introduction of the head; and
a. single piece pressure element (6, 6', 6") located at least partly in the accommodation space, the pressure element having a top end (61) facing the top end of the receiving part and a bottom end (62) facing the bottom end of the receiving part and a flexible portion to clamp the head;
wherein the flexible portion comprises at least one first slit (70, 700, 701) **characterized in that** the at least one first slit (70, 700, 701) is arranged at a distance from the bottom end of the pressure element, the first slit extending partially around the central axis (M) when the pressure element is in the receiving part body (5), and **in that** at least one second slit (73, 703, 704) is provided extending from the bottom end (62) of the pressure element into the first slit (70, 700, 701).

2. The receiving part of claim 1, wherein by the at least one first slit (70, 700, 701) and the at least one second slit (73, 703, 704) a slit ring (71, 71') is formed.

3. The receiving part of claim 2, wherein the slit ring (71, 71') is connected to the pressure element via at least one connecting strip (72, 702a, 702b) in a monolithic manner and is configured to laterally encompass the head.

4. The receiving part of claims 2 or 3, wherein the slit ring (71, 71') has an exterior tapered surface portion that tapers towards the bottom end (62) of the pressure element (6, 6', 6").

5. The receiving part of one of claims 1 to 4, wherein the at least one second slit (73, 703, 704) extends substantially coaxially to the central axis (M).

6. The receiving part of one of claims 1 to 5, wherein the at least one first slit (70, 700, 701) is substantially transverse to the central axis (M) and preferably perpendicular to the central axis (M).

7. The receiving part of one of claims 1 to 6, wherein a single first slit (70) and a single second slit (73) are provided.

8. The receiving part of one of claims 1 to 6, wherein two first slits (700, 701) are provided and wherein two second slits (703, 704) are provided that are preferable arranged opposite to each other.

9. The receiving part of one of claims 1 to 8, wherein the at least one second slit (73) is arranged at one end of the at least one first slit (70) in a circumferential direction.

10. The receiving part of one of claims 1 to 9, wherein the flexible portion of the pressure element (6, 6', 6") has a hollow spherically-shaped inner surface portion (67) configured to accommodate the head (3).

11. The receiving part of one of claims 1 to 10, wherein the flexible portion is sized relative to the head such that the head is clamped by friction.

12. The receiving part of one of claims 1 to 11, wherein the pressure element (6, 6', 6") further comprises a substantially cylindrical portion (63, 63') with a recess (64) at its top end (61) that is configured to accommodate the rod (100).

13. The receiving part of one of claims 1 to 12, wherein a stop (8a, 69a, 8b, 69b; 80, 71, 670; 630a) is provided that prevents movement of the pressure element once it is arranged in the receiving part towards the top end of the receiving part.

14. The receiving part of one of claims 2 to 13, wherein the pressure element can assume in the receiving part body an inserting position in which the slit ring (71, 71') is allowed to expand laterally in the accomodation space so as to allow introduction of the head through the opening (16) at the bottom end (11).

15. The receiving part of one of claims 1 to 14, wherein the accommodation space is substantially cylindrical (12a) with a narrowing portion (12b) near the bottom end (11), the narrowing portion being preferably tapered or rounded.

16. The receiving part of claim 15, wherein the pressure element comprises a narrowing portion at its outer wall which is preferably tapered or rounded and which cooperates with the narrowing portion in the accommodation space.

17. A bone anchoring device comprising a receiving part according to one of claims 1 to 16 and a bone anchoring element (1) having a shaft (2) and a head (3) that preferably has a spherically-shaped outer surface portion and wherein preferably a closure element (7) is provided for securing the rod in the receiving part.

## Patentansprüche

1. Ein Aufnahmeteil zum Aufnehmen eines Stabs (100) zum Verbinden des Stabs mit einem Knochenverankerungselement (1), wobei das Aufnahmeteil aufweist:
einen Aufnahmeteilkörper (5, 5') mit einem oberen Ende (10), einem unteren Ende (11) und einer Mittenachse (M), die sich durch das obere Ende und das untere Ende hindurch erstreckt;
einen Kanal (13) zum Aufnehmen des Stabs, und
einen Aufnahmeraum (12a, 12b) zum Aufnehmen eines Kopfs (3) des Knochenverankerungselements, wobei der Aufnahmeraum eine Öffnung (16) an dem unteren Ende zum Einführen eines Kopfs besitzt; und
ein einstückiges Druckelement (6, 6', 6"), das zumindest teilweise in dem Aufnahmeraum angeordnet ist, wobei das Druckelement ein oberes Ende (61), das dem oberen Ende des Aufnahmeteils zugewandt ist, und ein unteres Ende (62), das dem unteren Ende des Aufnahmeteils zugewandt ist, und einen flexiblen Abschnitt besitzt, um den Kopf zu klemmen;
wobei der flexible Abschnitt wenigstens einen ersten Schlitz (70, 700, 701) umfasst,
**dadurch gekennzeichnet, dass** der wenigstens eine erste Schlitz (70, 700, 701) in einem Abstand von dem unteren Ende des Druckelements angeordnet ist, wobei sich der erste Schlitz teilweise um die Mittenachse (M) herum erstreckt, wenn sich das Druckelement in dem Aufnahmeteilkörper (5) befindet, und dass wenigstens ein zweiter Schlitz (73, 703, 704) vorgesehen ist, der sich von dem unteren Ende (62) des Druckelements in den ersten Schlitz (70, 700, 701) erstreckt.

2. Das Aufnahmeteil gemäß Anspruch 1, wobei der wenigstens eine erste Schlitz (70, 700, 701) und der wenigstens eine zweite Schlitz (73, 703, 704) einen Schlitzring (71, 71') ausbilden.

3. Das Aufnahmeteil gemäß Anspruch 2, wobei der Schlitzring (71, 71') mit dem Druckelement über wenigstens einen Verbindungsstreifen (72, 702a, 702b) in einstückiger Weise verbunden ist, und eingerichtet ist, seitlich den Kopf zu umfassen.

4. Das Aufnahmeteil gemäß Anspruch 2 oder 3, wobei der Schlitzring (71, 71') einen äußeren Verjüngungsoberflächenabschnitt besitzt, der sich zu dem unteren Ende (72) des Druckelements (6, 6', 6") verjüngt.

5. Das Aufnahmeteil gemäß einem der Ansprüche 1 bis 4, wobei sich der wenigstens eine zweite Schlitz (73, 703, 704) im Wesentlichen koaxial zur Mittenachse (M) erstreckt.

6. Das Aufnahmeteil gemäß einem der Ansprüche 1 bis 5, wobei sich der wenigstens eine erste Schlitz (70, 700, 701) im Wesentlichen quer zur Mittenachse (M) und vorzugsweise senkrecht zur Mittenachse (M) erstreckt.

7. Das Aufnahmeteil gemäß einem der Ansprüche 1 bis 6, wobei ein einzelner erster Schlitz (70) und ein einzelner zweiter Schlitz (73) vorgesehen sind.

8. Das Aufnahmeteil gemäß einem der Ansprüche 1 bis 6, wobei zwei erste Schlitze (700, 701) vorgesehen sind, und wobei zwei zweite Schlitze (703, 704) vorgesehen sind, die vorzugsweise entgegengesetzt zueinander angeordnet sind.

9. Das Aufnahmeteil gemäß einem der Ansprüche 1 bis 8, wobei der wenigstens eine zweite Schlitz (73) an einem Ende des wenigstens einen ersten Schlitzes (70) in einer Umfassungsrichtung angeordnet ist.

10. Das Aufnahmeteil gemäß einem der Ansprüche 1 bis 9, wobei der flexible Abschnitt des Druckelements (6, 6', 6") einen hohlen, sphärisch-geformten, inneren Oberflächenabschnitt (67) besitzt, der eingerichtet ist, den Kopf (3) aufzunehmen.

11. Das Aufnahmeteil gemäß einem der Ansprüche 1 bis 10, wobei der flexible Abschnitt relativ zu dem Kopf so dimensioniert ist, dass der Kopf durch Reibung geklemmt ist.

12. Das Aufnahmeteil gemäß einem der Ansprüche 1 bis 11, wobei das Druckelement (6, 6', 6") ferner einen im Wesentlichen zylindrischen Abschnitt (63, 63') mit einer Ausnehmung (64) an seinem oberen Ende (61) besitzt, der eingerichtet ist, den Stab (100) aufzunehmen.

13. Das Aufnahmeteil gemäß einem der Ansprüche 1 bis 12, wobei ein Anschlag (8a, 69a, 8b, 69b; 80, 71, 670; 630a) vorgesehen ist, welcher einer Bewegung des Druckelements zu dem ersten Ende des Aufnahmeteils hin vorbeugt, wenn es einmal in dem Aufnahmeteil angeordnet ist.

14. Das Aufnahmeteil gemäß einem der Ansprüche 2 bis 13, wobei das Druckelement in dem Aufnahmeteilkörper eine Einsetzposition einnehmen kann, in welchem es dem Schlitzring (71, 71') ermöglicht ist, seitlich in dem Aufnahmeraum so zu expandieren, dass das Einführen des Kopfs durch die Öffnung (16) an dem unteren Ende (11) ermöglicht wird.

15. Das Aufnahmeteil gemäß einem der Ansprüche 1 bis 14, wobei der Aufnahmeraum im Wesentlichen zylindrisch (12a) mit einem sich verengenden Abschnitt (12b) nahe dem unteren Ende (11) ist, wobei der sich verengende Abschnitt vorzugsweise sich verjüngend oder abgerundet ist.

16. Das Aufnahmeteil gemäß Anspruch 15, wobei das Druckelement einen sich verengenden Abschnitt an seiner Außenwand umfasst, welcher vorzugsweise sich verjüngend oder abgerundet ist, und welcher mit dem sich verengenden Abschnitt in dem Aufnahmeraum zusammenwirkt.

17. Eine Knochenverankerungsvorrichtung, umfassend ein Aufnahmeteil gemäß einem der Ansprüche 1 bis 16, und ein Knochenverankerungselement (1) mit einem Schaft (2) und einem Kopf (3), der vorzugsweise einen sphärisch-geformten äußeren Oberflächenabschnitt besitzt, und wobei vorzugsweise ein Verschlusselement (7) vorgesehen ist, um den Stab in dem Aufnahmeteil zu sichern.

## Revendications

1. Partie de réception pour la réception d'une tige (100) pour le couplage de la tige à un élément d'ancrage osseux (1), la partie de réception comportant
un corps de partie de réception (5, 5') ayant une extrémité supérieure (10), une extrémité inférieure (11) et un axe central (M) s'étendant à travers l'extrémité supérieure et l'extrémité inférieure ;
un canal (13) pour la réception de la tige, et
un espace de logement (12a, 12b) pour loger une tête (3) de l'élément d'ancrage osseux, l'espace de logement présentant une ouverture (16) au niveau de l'extrémité inférieure pour l'introduction de la tête ; et
un élément de pression en une seule pièce (6, 6', 6") situé au moins partiellement dans l'espace de logement, l'élément de pression présentant une extrémité supérieure (61) faisant face à l'extrémité supérieure de la partie de réception et une extrémité inférieure (62) faisant face à l'extrémité inférieure de la partie de réception et une portion flexible pour serrer la tête ;
dans laquelle la portion flexible comprend au moins une première fente (70, 700, 701),
**caractérisé en ce que** l'au moins une première fente (70, 700, 701) est agencée à une distance par rapport à l'extrémité inférieure de l'élément de pression, la première fente s'étendant partiellement autour de l'axe central (M) lorsque l'élément de pression est dans le corps de partie de réception (5), et **en ce qu'**au moins une deuxième fente (73, 703, 704) est prévue de manière à s'étendre à partir de l'extrémité inférieure (62) de l'élément de pression dans la première fente (70, 700, 701).

2. Partie de réception de la revendication 1, dans laquelle par l'au moins une première fente (70, 700, 701) et l'au moins une deuxième fente (73, 703, 704) un anneau à fente (71, 71') est formé.

3. Partie de réception de la revendication 2, dans laquelle l'anneau à fente (71, 71') est relié à l'élément de pression par l'intermédiaire d'au moins une bande de liaison (72, 702a, 702b) d'une manière monolithique et est configuré pour englober latéralement la tête.

4. Partie de réception de la revendication 2 ou 3, dans laquelle l'anneau à fente (71, 71') présente une portion de surface effilée extérieure qui s'effile vers l'extrémité inférieure (62) de l'élément de pression (6, 6' 6'').

5. Partie de réception de l'une des revendications 1 à 4, dans laquelle l'au moins une deuxième fente (73, 703, 704) s'étend de manière substantiellement coaxiale à l'axe central (M).

6. Partie de réception de l'une des revendications 1 à 5, dans laquelle l'au moins une première fente (70, 700, 701) est substantiellement transversale à l'axe central (M) et de préférence perpendiculaire à l'axe central (M).

7. Partie de réception de l'une des revendications 1 à 6, dans laquelle une seule première fente (70) et une seule deuxième fente (73) sont prévues.

8. Partie de réception de l'une des revendications 1 à 6, dans laquelle deux premières fentes (700, 701) sont prévues et dans laquelle deux deuxièmes fentes (703, 704), qui sont de préférence agencées de manière opposée l'une à l'autre, sont prévues.

9. Partie de réception de l'une des revendications 1 à 8, dans laquelle l'au moins une deuxième fente (73) est agencée au niveau d'une extrémité de l'au moins une première fente (70) dans une direction circonférentielle.

10. Partie de réception de l'une des revendications 1 à 9, dans laquelle la portion flexible de l'élément de pression (6, 6', 6'') présente une portion de surface intérieure de forme sphérique creuse (67) configurée pour loger la tête (3).

11. Partie de réception de l'une des revendications 1 à 10, dans laquelle la portion flexible est dimensionnée par rapport à la tête de sorte que la tête soit serrée par friction.

12. Partie de réception de l'une des revendications 1 à 11, dans laquelle l'élément de pression (6, 6', 6'') comprend en outre une portion substantiellement cylindrique (63, 63') avec un évidement (64) au niveau de son extrémité supérieure (61) qui est configuré pour loger la tige (100).

13. Partie de réception de l'une des revendications 1 à 12, dans laquelle une butée (8a, 69a, 8b, 69b, 80, 71, 670, 630a), qui empêche le déplacement de l'élément de pression une fois qu'il est agencé dans la partie de réception vers l'extrémité supérieure de la partie de réception, est prévue.

14. Partie de réception de l'une des revendications 2 à 13, dans laquelle l'élément de pression peut adopter, dans le corps de partie de réception, une position d'insertion dans laquelle l'anneau à fente (71, 71') est autorisé à s'élargir latéralement dans l'espace de logement de manière à permettre l'introduction de la tête à travers l'ouverture (16) au niveau de l'extrémité inférieure (11).

15. Partie de réception de l'une des revendications 1 à 14, dans laquelle l'espace de logement est substantiellement cylindrique (12a) avec une portion de rétrécissement (12b) proche de l'extrémité inférieure (11), la portion de rétrécissement étant de préférence effilée ou arrondie.

16. Partie de réception de la revendication 15, dans laquelle l'élément de pression comprend une portion de rétrécissement au niveau de sa paroi extérieure qui est de préférence effilée ou arrondie et qui coopère avec la portion de rétrécissement dans l'espace de logement.

17. Dispositif d'ancrage osseux comprenant une partie de réception selon l'une des revendications 1 à 16 et un élément d'ancrage osseux (1) présentant un arbre (2) et une tête (3) qui présente de préférence une portion de surface extérieure de forme sphérique et dans lequel de préférence un élément de fermeture (7) est prévu pour fixer la tige dans la partie de réception.
